# FASCICULE DE BREVET EUROPEEN

(11) **EP 2 939 003 B1**
(45) Date de publication et mention de la délivrance du brevet: **07.08.2019**
(21) Numéro de dépôt: 13824627.7
(22) Date de dépôt: 23.12.2013
(51) Int. Cl.: G01N 21/27, G01N 21/64, G01N 21/77

(54) **PROCÉDÉ ET SYSTÈME POUR DÉTECTER ET MESURER DES SIGNAUX DE FLUORESCENCE**
VERFAHREN UND SYSTEM ZUR ERKENNUNG UND MESSUNG VON FLUORESZENZSIGNALEN
METHOD AND SYSTEM FOR DETECTING AND MEASURING FLUORESCENCE SIGNALS

(30) Priorité: 26.12.2012 FR 1262790
(43) Date de publication de la demande: 04.11.2015
(73) Titulaire: Biomérieux, 69280 Marcy l'Étoile (FR)
(72) Inventeur: COLIN, Bruno, F-69280 Marcy l'Etoile (FR); DRAZEK, Laurent, F-38100 Grenoble (FR); PINSTON, Frédéric, F-38000 Grenoble (FR)
(74) Mandataire: Schoen, Elsa Imilia
(86) Numéro de dépôt international: PCT/FR2013/053258
(87) Numéro de publication internationale: WO 2014/102502

(56) Documents cités:
- EP-A2- 1 253 420
- WO-A1-2012/022963
- DE-A1- 10 344 140
- FR-A1- 2 887 626

## Description

### Domaine technique

La présente invention concerne, de manière générale, un procédé et un système pour détecter et mesurer des signaux de fluorescence émis par un milieu de réaction, issu d'un échantillon à tester afin de procéder à une analyse dudit échantillon susceptible de contenir des analytes. Les analytes peuvent être représentatifs de la présence de micro-organismes ou d'une maladie que l'on souhaite détecter, caractériser ou suivre.

### Etat de la technique

Dans le domaine de l'analyse d'échantillons susceptibles de contenir des analytes d'intérêt, il est connu d'utiliser des méthodes basées sur des mesures fluorimétriques, c'est-à-dire des mesures permettant de quantifier des signaux de fluorescence émis au cours de l'analyse biologique de l'échantillon. L'analyse de l'échantillon à tester doit donc comprendre la mise en oeuvre d'un réactif, représentatif de l'analyte à détecter ou à quantifier dans l'échantillon. Le réactif permet d'obtenir un produit de réaction qui possède des propriétés de fluorescence. En effet, en application du principe de fluorescence, un tel produit de réaction exposé à une source lumineuse, correspondant à une première longueur d'onde dite d'excitation, émet à son tour des rayons lumineux selon une deuxième longueur d'onde dite d'émission. Le milieu issu de l'échantillon à tester et contenant le produit de réaction est alors un milieu de réaction. Dans ce milieu de réaction, la détection des signaux de fluorescence, associée à un traitement du signal de ces signaux de fluorescence, permet de déterminer, par exemple, la présence ou la concentration de l'analyte spécifique recherché au sein de l'échantillon à tester.

Afin d'obtenir une valeur fiable de la concentration de l'analyte spécifique, la détection des signaux de fluorescence doit être réalisée avec précision. Or, lors d'une telle détection, des sources d'interférences telles que des particules de matière proche du milieu de réaction, issu de l'échantillon et comprenant le produit de réaction, peuvent produire des signaux de fluorescence parasites.

Le document US 2007/0154938 décrit une méthode pour analyser un échantillon biologique, localisé sur une plaque, à l'aide de mesures de signaux de fluorescence. Ainsi, une source lumineuse illumine le milieu de réaction issu de l'échantillon biologique afin d'obtenir une émission de signaux de fluorescence en provenance de cet échantillon biologique. Selon le document US 2007/0154938, les émissions de signaux de fluorescence en provenance du milieu de réaction issu de l'échantillon biologique sont parasitées par l'émission de signaux de fluorescence en provenance de substances étrangères localisées sur la plaque, à proximité du milieu de réaction.

Le document US 2007/0154938 propose de résoudre ce problème de parasitage de signal de fluorescence en évitant de prendre en compte des données de fluorescence associées à la localisation des substances étrangères sur la plaque. Ainsi, une valeur moyenne des signaux de fluorescence émis par le milieu de réaction est calculée en considérant seulement les signaux de fluorescence associés à la localisation du milieu de réaction sur la plaque. La détection et la mesure des signaux de fluorescence émis par le milieu de réaction sont donc plus précises. L'analyse de l'échantillon biologique est donc améliorée.

Le document EP 1 253 420 A2 divulgue un procédé de calibrage pour un analyseur d'échantillons sanguins afin de déterminer les paramètres spécifiques au sang tel que le taux d'hématocrites. Le procédé selon D1 comprend une étape de calcul afin de supprimer les émissions de fluorescence parasites produites par une cuvette.

Cependant, dans certaines situations, le matériau de la cuvette contenant le milieu de réaction est également capable d'émettre des signaux de fluorescence en réponse à une illumination. Ces signaux de fluorescence sont également considérés comme des signaux parasites vis-à-vis des signaux de fluorescence émis par le milieu de réaction. La suppression de ces signaux parasites s'avère complexe dans la mesure où le matériau de la cuvette ne peut être distingué du milieu de réaction lors de la détection et de la mesure des signaux de fluorescence.

### Exposé de l'invention

La présente invention vise à surmonter au moins partiellement les problèmes mentionnés ci-dessus.

Un premier objectif de l'invention consiste à fournir un procédé d'analyse d'un échantillon à tester pour la détermination de la présence ou la quantification d'un analyte susceptible d'être présent dans ledit échantillon, tel que défini dans la revendication 1.

Selon un mode de réalisation de l'invention, l'illumination de la cuvette comprend une illumination dans l'air, c'est-à-dire avant la première bordure de la paroi de la cuvette, sur la première bordure de la paroi de la cuvette, sur la paroi de la cuvette, sur la seconde bordure de la paroi de la cuvette et dans l'air, c'est-à-dire après la seconde bordure de la paroi de la cuvette.

Selon un mode de réalisation de l'invention, la détection d'un signal de fluorescence comprend la détection d'un signal de fluorescence en provenance d'une deuxième surface S2 de la cuvette, opposée à la première surface S1.

Selon un mode de réalisation de l'invention, le signal résultant correspond à un signal de fluorescence émis par l'analyte.

Selon un mode de réalisation de l'invention, le signal résultant correspond à un signal de fluorescence émis par la cuvette.

Selon un mode de réalisation de l'invention, l'intensité du signal résultant est proportionnelle ou inversement proportionnelle à la concentration de l'analyte dans l'échantillon testé.

Selon un mode de réalisation de l'invention, la source lumineuse comprend une diode électroluminescente (LED).

Selon un mode de réalisation de l'invention, la réaction est une réaction antigène-anticorps, en particulier par dosage immuno-enzymatique.

Selon un mode de réalisation de l'invention, l'étape de réalisation d'une opération de calcul comprend la réalisation d'une opération de déconvolution du signal résultant par le profil d'éclairement de la source lumineuse pour produire un signal résultant correspondant à l'émission du signal de fluorescence produit uniquement par le milieu de réaction.

Un autre objectif de l'invention consiste à fournir un système d'analyse d'un échantillon à tester pour la détermination de la présence ou la quantification d'un analyte susceptible d'être présent dans ledit échantillon, tel que défini dans la revendication 9.

Selon un mode de réalisation de l'invention, le dispositif optique illumine la première surface S1 de la cuvette.

Selon un mode de réalisation de l'invention, de l'invention le dispositif optique détecte les émissions de fluorescence en provenance d'une deuxième surface S2 de la cuvette, opposée à la première surface S1.

### Brève description des dessins

L'invention, sa fonctionnalité, ses applications ainsi que ses avantages seront mieux appréhendés à la lecture de la présente description, faite en référence aux figures, dans lesquelles :
- la figure 1 montre un dispositif d'analyse pour procéder à des analyses biologiques, selon un mode de réalisation de l'invention, à titre d'exemple ;
- la figure 2 montre une barrette d'analyse et un cône d'analyse associés pour une analyse biologique, selon un mode de réalisation de l'invention, à titre d'exemple ;
- la figure 3 montre une vue en perspective détaillée d'une dernière cuvette de la barrette d'analyse selon la figure 2, selon un mode de réalisation de l'invention, à titre d'exemple ;
- la figure 4 montre une première vue latérale de la dernière cuvette selon la figure 3, définissant une première surface S1, selon un mode de réalisation de l'invention, à titre d'exemple ;
- la figure 5 montre une deuxième vue latérale de la dernière cuvette selon la figure 3, définissant une deuxième surface S2, selon un mode de réalisation de l'invention, à titre d'exemple ;
- la figure 6 montre la barrette d'analyse, selon la figure 2, positionnée au sein du dispositif d'analyse selon la figure 1 et un dispositif optique à proximité de la barrette d'analyse, selon un mode de réalisation de l'invention, à titre d'exemple ;
- la figure 7 montre une vue en coupe du dispositif optique selon la figure 6, selon un mode de réalisation de l'invention, à titre d'exemple ;
- la figure 8 montre les différentes positions du dispositif optique selon les figures 6 et 7, lors de l'étape d'illumination du milieu de réaction localisé dans la dernière cuvette selon les figures 2 et 3, selon un mode de réalisation de l'invention, à titre d'exemple ;
- la figure 9 montre une vue de face d'une surface de la dernière cuvette de la barrette d'analyse avec une représentation schématique de la forme des différents faisceaux d'excitation sur cette surface lors de l'étape d'illumination du milieu de réaction, selon un mode de réalisation de l'invention, à titre d'exemple ;
- la figure 10 montre les différentes étapes d'un procédé selon un mode de réalisation de l'invention, à titre d'exemple ;
- la figure 11 montre deux représentations graphiques relatives à l'intensité des signaux de fluorescence concernant deux dernières cuvettes de deux barrettes d'analyse adjacentes, les signaux de fluorescence étant émis par une surface d'une dernière cuvette et par le milieu de réaction, en fonction de la localisation du dispositif optique émettant les faisceaux d'excitation, selon un mode de réalisation de l'invention, à titre d'exemple ;
- la figure 12 montre un graphe représentant un profil du signal d'éclairement d'une source lumineuse telle qu'une diode électroluminescente (LED), selon un mode de réalisation de l'invention, à titre d'exemple ;
- la figure 13 montre un graphe représentant un profil du signal de fluorescence émis par une paroi de la dernière cuvette selon la figure 3, lorsque cette paroi est éclairée par une source lumineuse ponctuelle, selon un mode de réalisation de l'invention, à titre d'exemple.

### Description détaillée de l'invention

La description détaillée ci-après a pour but d'exposer l'invention de manière suffisamment claire et complète, notamment à l'aide d'exemples, mais ne doit en aucun cas être regardée comme limitant l'étendue de la protection aux modes de réalisation particuliers et aux exemples présentés ci-après. Les exemples ci-après permettront de mieux appréhender la présente invention. Toutefois, ces exemples ne sont donnés qu'à titre illustratif et ne doivent en aucun cas être regardés comme limitant la portée de ladite invention d'une quelconque manière.

Le terme signal ou faisceau est utilisé au singulier ci-après. Cependant, la présente invention concerne un ensemble de signaux de fluorescence et un ensemble de faisceaux d'excitation.

La présente invention concerne l'analyse d'échantillons. Selon la présente invention, l'échantillon peut être de diverses origines, par exemple d'origine alimentaire, environnementale, vétérinaire, clinique, pharmaceutique ou cosmétique.

Parmi les échantillons d'origine alimentaire, l'on peut citer, de façon non-exhaustive, un échantillon de produits lactés (yaourts, fromages...), de viande, de poisson, d'oeuf, de fruit, de légume, d'eau, de boisson (lait, jus de fruits, soda, etc.). Bien évidemment, ces échantillons d'origine alimentaire peuvent aussi provenir de sauces ou de plats plus élaborés ou des matières premières non transformées ou partiellement transformées. Un échantillon alimentaire peut également être issu d'une alimentation destinée aux animaux, telle que des tourteaux, des farines animales.

Tel qu'indiqué précédemment, l'échantillon biologique peut être d'origine environnementale et peut consister, par exemple, en un prélèvement de surface, d'eau, etc.

L'échantillon peut également consister en un échantillon biologique, d'origine clinique, humaine ou animale, pouvant correspondre à des prélèvements de fluide biologique (urine, sang total, ou dérivés tel que sérum, plasma, salive, pus, liquide céphalo-rachidien, etc.), de selles (par exemple diarrhées cholériques), de prélèvements de nez, de gorge, de peau, de plaies, d'organes, de tissus ou de cellules isolées. Cette liste n'est évidemment pas exhaustive.

D'une manière générale, le terme « échantillon » se réfère à une partie ou à une quantité, plus particulièrement une petite partie ou une petite quantité, prélevée à partir d'une ou plusieurs entités aux fins d'analyse. Cet échantillon peut éventuellement avoir subi un traitement préalable, incluant par exemple des étapes de mélange, de dilution ou encore de broyage, en particulier si l'entité de départ est à l'état solide.

L'échantillon analysé est susceptible de - ou suspecté de - contenir au moins un analyte représentatif de la présence de microorganismes ou d'une maladie à détecter, caractériser ou suivre. L'analyse d'échantillons met en oeuvre une réaction entre l'analyte d'intérêt et un ou des partenaire(s) de liaison spécifique(s) à l'analyte.

Selon un mode réalisation de la présente invention, la réaction est une réaction immunologique qui implique comme partenaire(s) de liaison des antigènes et/ou des anticorps, des récepteurs à l'analyte et l'analyte à déterminer est une protéine, un peptide ou un haptène. A titre d'exemples de telles réactions, on peut citer les réactions dites « de compétition » et les réactions dites « sandwich » mises en oeuvre dans des dosages immuno-enzymatiques de type ELISA ou ELFA.

Bien entendu, le terme « immuno » dans « immunodosage » n'est pas à considérer dans la présente demande comme indiquant strictement que le partenaire de liaison est un partenaire immunologique, tel qu'un anticorps. En effet, l'Homme du Métier utilise également largement ce terme lorsque le partenaire de liaison, aussi appelé ligand, n'est pas un partenaire immunologique mais est par exemple un récepteur à l'analyte que l'on veut doser. Ainsi, il est connu de parler du dosage ELISA (Enzyme-Linked Immunosorbent Assay) pour des dosages qui utilisent des partenaires de liaison non immunologiques, appelés plus largement en anglais « Ligand Binding Assay », que l'on pourrait traduire par « Dosage utilisant la liaison à un ligand », alors que le terme même « immuno » est inclus dans l'acronyme ELISA. Par souci de clarté, la Demanderesse utilisera dans la demande le terme « immuno » pour tout dosage d'un analyte protéique, même quand il n'est pas un partenaire immunologique.

Selon un autre mode de réalisation de la présente invention, la réaction est une réaction d'hybridation et l'analyte à déterminer est un acide nucléique de type ADN ou ARN, à savoir une réaction qui implique un fragment nucléotidique complémentaire de l'analyte à déterminer.

Bien entendu, la réaction mise en oeuvre lors de l'analyse de l'échantillon à tester utilise un réactif qui, en présence de l'analyte à détecter ou quantifier, est capable de générer directement ou indirectement la fluorescence. Ce réactif permet donc d'obtenir un produit de réaction possédant des propriétés de fluorescence. Le produit de réaction est alors contenu dans le milieu de réaction qui est issu de l'échantillon mais dont le contenu est différent.

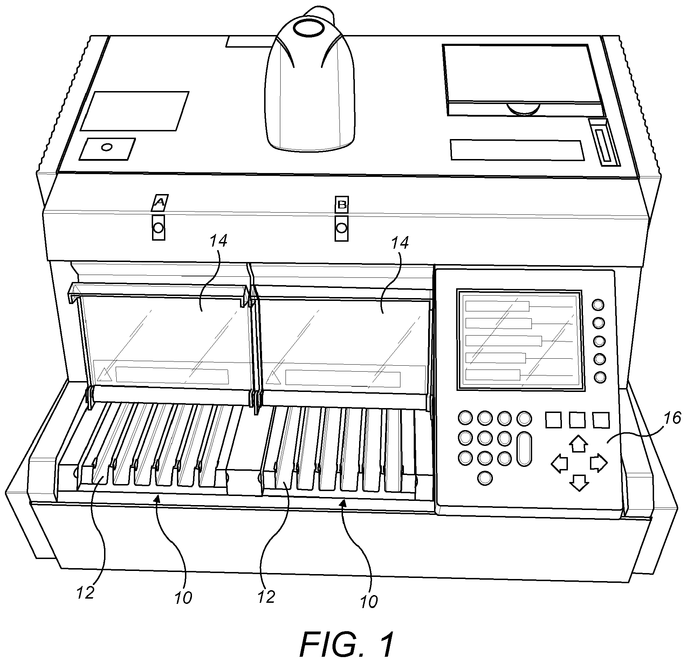

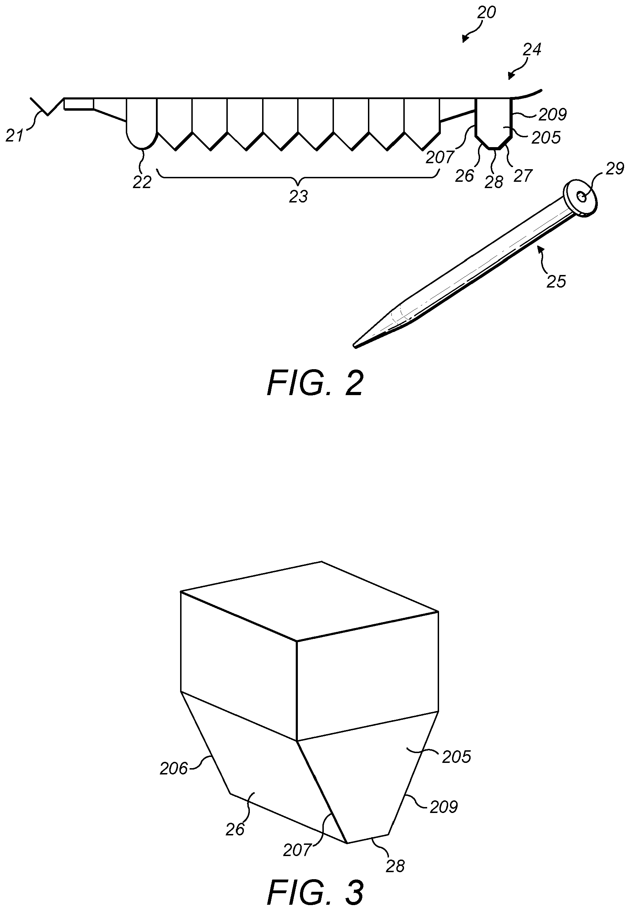

La figure 1 montre un dispositif d'analyse 1 pour procéder à des analyses biologiques d'échantillons. Ce dispositif d'analyse 1 comprend au moins un réceptacle 10 composé de plusieurs rails 12. Un capot mobile de protection 14 est associé à chaque réceptacle 10. Chaque réceptacle 10 comprend un nombre de rails 12 variable, par exemple compris entre 1 et 10. La figure 1 montre par exemple 6 rails. Chaque rail 12 peut recevoir une barrette d'analyse 20 comme décrit ci-après sur la figure 2. Un rail 12 s'étend depuis l'extérieur du dispositif d'analyse 1 jusqu'à l'intérieur du dispositif d'analyse 1.

Le dispositif d'analyse 1 comprend également une interface utilisateur 16 afin de lancer le programme relatif à l'analyse souhaitée.

Comme montré sur la figure 2, la barrette d'analyse 20 est d'une longueur d'environ 15 cm, supérieure à la longueur d'un rail 12. La barrette d'analyse 20 comprend un moyen de préhension 21 afin de faciliter l'insertion de la barrette d'analyse 20 par un utilisateur au sein d'un rail 12 du dispositif d'analyse 1. La barrette d'analyse 20 comprend également plusieurs cuvettes. Un film protecteur (non montré) localisé sur les rebords supérieurs des cuvettes permet de recouvrir de manière étanche les différents contenus des différentes cuvettes. Ainsi, la barrette d'analyse 20 comprend une première cuvette 22 localisée à une première extrémité de la barrette d'analyse 20. La première cuvette 22 comprend par exemple une paroi de forme oblongue. La première cuvette 22 contient l'échantillon à analyser. Une pluralité de cuvettes 23 est disposée après la première cuvette 22. Les cuvettes 23 comprennent des parois verticales réunies par une base de forme pyramidale, avec quatre faces triangulaires orientées deux à deux en vis-à-vis. Les cuvettes 23 peuvent également comprendre une paroi continue de forme oblongue. Chaque cuvette 23 contient une solution. Les cuvettes 23 comprennent des réactifs nécessaires à la réaction pour déterminer la présence de l'analyte à tester ou quantifier l'analyte à tester. En particulier, l'une des cuvettes 23 comprend un réactif qui, en présence de l'analyte à détecter ou à quantifier, est capable de générer un produit de réaction possédant des propriétés de fluorescence si l'analyte est présent dans ledit échantillon à tester. L'analyse de l'échantillon est basée sur ce produit de réaction qui se trouve dans le milieu de réaction.

Comme montré sur les figures 2 et 3, la barrette d'analyse 20 comprend une dernière cuvette 24 localisée à la deuxième extrémité de la barrette d'analyse 20. La dernière cuvette 24 comprend des parois verticales 204 (non montrée) et 205 qui possèdent une épaisseur de l'ordre du millimètre. Chaque paroi verticale 204, 205 est délimitée par des bordures respectives 206, 207, 208, 209 comme montré sur les figures 3, 4, 5, 8, 9 et 11. Ces bordures 206, 207, 208, 209 comprennent également une épaisseur de l'ordre du millimètre, supérieure à l'épaisseur des parois 26 et 27. Les parois verticales 204 et 205 sont réunies par une base comprenant deux parois inclinées 26 et 27 jointes par une paroi étroite horizontale 28 comme montré sur les figures 2 et 3. Les parois 26 et 27 sont délimitées par les parois verticales 204 et 205. Ainsi, la paroi 26 est délimitée par les bordures 206 et 207. La paroi 27 est délimitée par les bordures 208 et 209. A la fin d'un cycle d'analyse, la dernière cuvette 24 contient un milieu de réaction comprenant différents liquides prélevés dans les cuvettes 22 et 23.

Le transport de liquide entre les différentes cuvettes, 22, 23 et 24 est effectué à l'aide d'un cône d'analyse 25 montré sur la figure 2. Le cône d'analyse 25 sert de phase solide pour la réaction. En effet, le cône d'analyse 25 est recouvert d'au moins un partenaire de liaison à l'analyte. Le cône d'analyse 25 permet aussi d'aspirer du liquide depuis la première cuvette 22 et de refouler le liquide aspiré à l'intérieur d'une autre cuvette 23 pour obtenir un premier mélange. Puis le cône d'analyse 25 aspire ce premier mélange et refoule le premier mélange au sein d'une autre cuvette 23 et ainsi de suite jusqu'à la dernière cuvette 24 dans laquelle le milieu de réaction est refoulé. Selon le type d'analyse souhaitée, toutes ou certaines cuvettes 23 peuvent être nécessaires à l'analyse du liquide concernant l'échantillon initialement prélevé dans la cuvette 22.

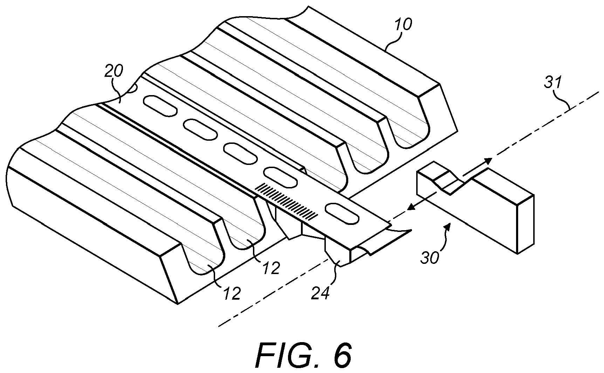

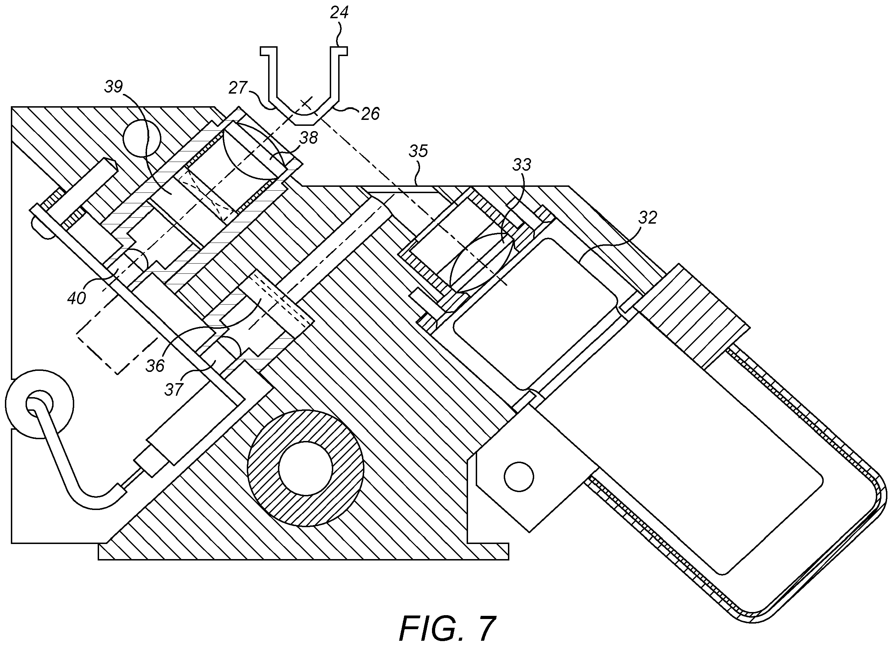

Lorsque la barrette d'analyse 20 est insérée sur un rail 12, seule la dernière cuvette 24 dépasse du rail 12 afin de permettre à un dispositif optique 30 montré sur les figures 6 et 7 de se positionner pour procéder à l'analyse du milieu de réaction contenu dans la dernière cuvette 24.

L'analyse de l'échantillon à tester est basée sur une étape d'illumination et une étape de détection comme décrit ci-après au moyen du dispositif optique 30 montré sur les figures 6 et 7 et détaillé ci-après.

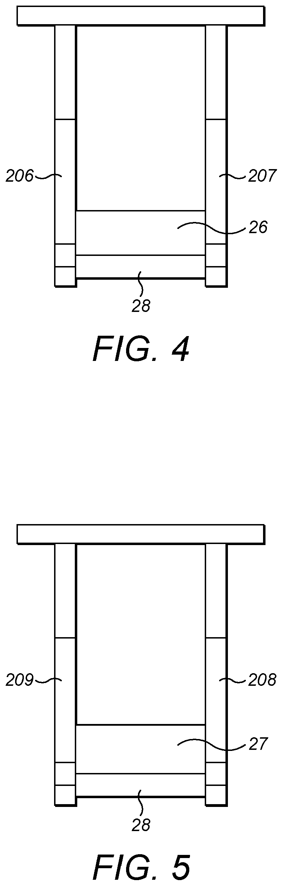

L'analyse de l'échantillon à tester est réalisée en utilisant seulement le contenu de la dernière cuvette 24. Ainsi, l'illumination du contenu de la dernière cuvette 24 est réalisée sur une première surface S1, montrée sur la figure 4, de la dernière cuvette 24. La première surface S1 comprend la paroi inclinée 26 et les bordures 206 et 207 de la dernière cuvette 24. La détection des signaux de fluorescence émis par la dernière cuvette 24 est réalisée en considérant la lumière émise au travers d'une deuxième surface S2, montrée sur la figure 5, de la dernière cuvette 24. La surface S2 comprend la paroi inclinée 27 et les bordures 208 et 209. La surface S2 est opposée à la surface S1. La deuxième surface S2 permet de détecter les signaux de fluorescence émis lors de l'illumination de la première surface S1.

La barrette d'analyse 20 est réalisée dans un matériau plastique spécifique tel que du polypropylène. Ainsi, les cuvettes 22, 23 et 24 sont également réalisées dans ce matériau plastique spécifique. Le matériau plastique spécifique permet de conserver efficacement les liquides et autres matières nécessaires à toute analyse biologique. Ce matériau plastique spécifique possède des propriétés physico-chimiques telles que la fluorescence. Ainsi, lorsque la dernière cuvette 24 subit une illumination sur la première surface S1 comprenant la paroi 26 et les bordures 206 et 207, cette première surface S1 émet des signaux de fluorescence.

Toute autre surface de la dernière cuvette 24 ayant reçu des signaux d'excitation émet également des signaux de fluorescence.

Comme montré sur la figure 6, le dispositif optique 30 est situé à l'intérieur du dispositif d'analyse 1, sur le même plan horizontal que les différents rails 12. Le dispositif optique 30 peut coulisser sur un chemin 31 dont la direction est perpendiculaire à la direction des rails 12. Comme montré sur la figure 6, la forme du dispositif optique 30 est telle que le dispositif optique 30 est mis en regard des parois 26 et 27 de la dernière cuvette 24.

La figure 7 montre les différents éléments localisés à l'intérieur du dispositif optique 30.

Le dispositif optique 30 comprend une source lumineuse 32 telle qu'une diode électroluminescente (LED) qui émet des signaux lumineux ou des faisceaux d'excitation. La source lumineuse 32 comprend toute source monochromatique correspondant à la longueur d'onde du pic d'excitation de la molécule chimique utilisée en tant que marqueur, telle qu'une longueur d'onde de 370 nm pour la 4-méthylumbelliférone. Le faisceau de la source lumineuse est de l'ordre de l'épaisseur de la paroi et peut être légèrement inférieur à l'épaisseur de la paroi. Par exemple, la largeur du faisceau est de l'ordre du millimètre. La source lumineuse 32 produit par exemple 200 brèves illuminations ou éclairs espacés de 100ms pendant le déplacement du dispositif optique 30 le long du chemin 31. Lors du fonctionnement du dispositif optique 30, la source lumineuse 32 éclaire la paroi 26 de la dernière cuvette 24. Dans la mesure où le dispositif optique 30 est mobile sur le chemin 31, la source lumineuse 32 peut donc émettre plusieurs faisceaux d'excitation illuminant différentes zones de la première surface S1 comprenant les parois 26, 206, 207. Les différentes zones illuminées correspondent aux différentes positions du dispositif optique 30 sur le chemin 31.

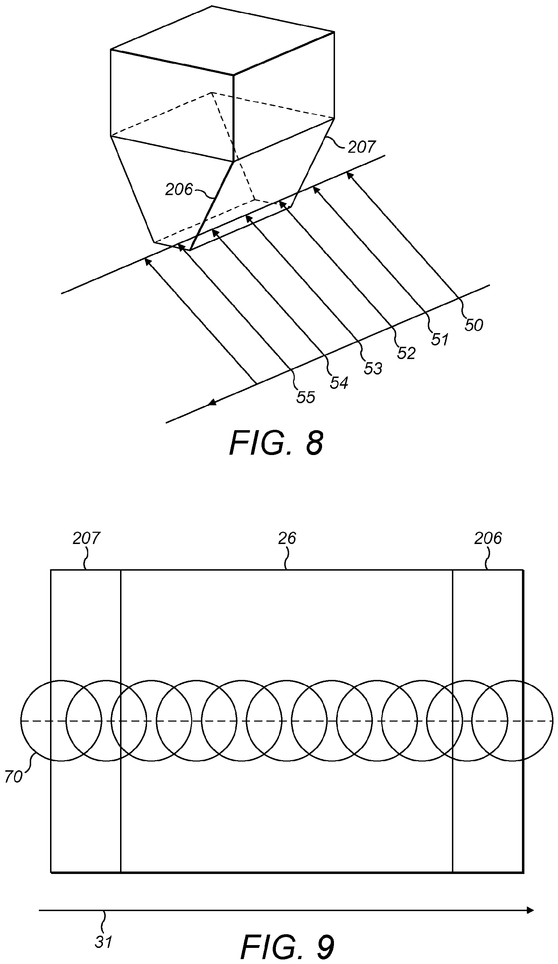

Devant la première surface S1, la source lumineuse 32 produit une série d'émissions de faisceaux d'excitation pendant le déplacement du dispositif optique 30. Ainsi, l'émission des faisceaux d'excitation se produit successivement, pour une première surface S1 spécifique comme montré sur la figure 8 avec les positions référencées de 50 à 55. Chaque émission d'un signal d'excitation peut être catégorisée comme suit :
1) émission dans l'air, c'est-à-dire avant la première bordure 207 de la paroi 26;
2) sur la première bordure 207 de la paroi 26 ;
3) sur la paroi 26 ;
4) sur la seconde bordure 206 de la paroi 26 ;
5) dans l'air, c'est-à-dire après la deuxième bordure 206 de la paroi 26.

Ainsi, le dispositif optique 30 peut se déplacer le long du chemin 31 afin d'opérer sur les différentes dernières cuvettes 24 de chaque barrette d'analyse 20 présente dans les différents rails 12 au sein des différents réceptacles 10.

Un dispositif électronique de contrôle (non montré), tel qu'un ordinateur, localisé dans le dispositif d'analyse 1 permet de contrôler la source lumineuse 32 afin d'émettre des faisceaux d'excitation selon une fréquence et une intensité prédéterminées. Le dispositif électronique de contrôle comprend une source d'alimentation (non montrée) et un circuit de commande (non montré) afin de déclencher l'émission des faisceaux d'excitation.

Le dispositif électronique de contrôle calcule la valeur moyenne des valeurs relatives à la prise de mesure des signaux de fluorescence détectés. Ainsi, le dispositif électronique de contrôle peut compenser les éventuelles variations d'intensité de la source lumineuse 32, dues par exemple au vieillissement de la source lumineuse.

Le dispositif optique 30 comprend également un élément optique 33 tel qu'une lentille asphérique 33 pour projeter les faisceaux d'excitation sur la paroi 26.

Le dispositif optique 30 comprend un séparateur optique 35 tel qu'un miroir semi-argenté afin de réfléchir le signal d'excitation en provenance de la source lumineuse 32.

Le dispositif optique 30 comprend également un filtre ultra-violet (UV) 36 afin de filtrer les rayons UV lors de la réflexion du faisceau d'excitation. Le filtre UV 36 est associé à une photodiode de référence 37 qui reçoit le signal réfléchi après la transmission au travers du filtre UV 36. Ainsi, la photodiode de référence 37 produit un signal électrique proportionnel à l'intensité lumineuse du faisceau d'excitation.

Le dispositif optique 30 comprend également une lentille asphérique 38 centrée sur le centre de la dernière cuvette 24 et positionnée selon un angle de 90° par rapport à la direction du faisceau d'excitation. Ainsi, la lentille asphérique 38 peut recevoir les signaux de fluorescence émis, notamment, par le matériau et le contenu de la dernière cuvette 24. La deuxième surface S2 de la dernière cuvette 24 émet également des signaux de fluorescence, en réaction à l'illumination de la première surface S1 par la source lumineuse 32.

Le dispositif optique 30 comprend également un filtre à bande passante étroite 39 afin de limiter la détection à un signal de fluorescence dont la longueur d'onde est de 450nm par exemple.

Le dispositif optique 30 comprend également un moyen de détection 40 tel qu'une photodiode à haute sensibilité afin de détecter la quantité de fluorescence émise par l'échantillon à tester contenu dans la dernière cuvette 24, et par la deuxième surface S2 comprenant la paroi 27 de la dernière cuvette 24 et les bordures 208 et 209. Comme montré sur la figure 9, la source lumineuse 32 peut émettre des faisceaux d'excitation de forme circulaire ou sensiblement circulaire comme montré par la forme circulaire 70. Lors de l'illumination d'une dernière cuvette 24 donnée, la source lumineuse 32 du dispositif optique 30 émet plusieurs faisceaux d'excitation localisés sur la première surface S1 comprenant la bordure 206, la paroi 26, la bordure 207 d'une dernière cuvette 24. Les faisceaux d'excitation présentent des zones d'intersection afin de garantir une illumination de la surface S1 sur toute la longueur de cette surface S1.

Lors du déplacement du dispositif optique 30, la source lumineuse 32 émet simultanément en dynamique les faisceaux d'excitation afin de balayer l'ensemble des premières surfaces S1 comprenant la bordure 206, la paroi 26 et la bordure 207 des dernières cuvettes 24 pour chaque barrette d'analyse 20.

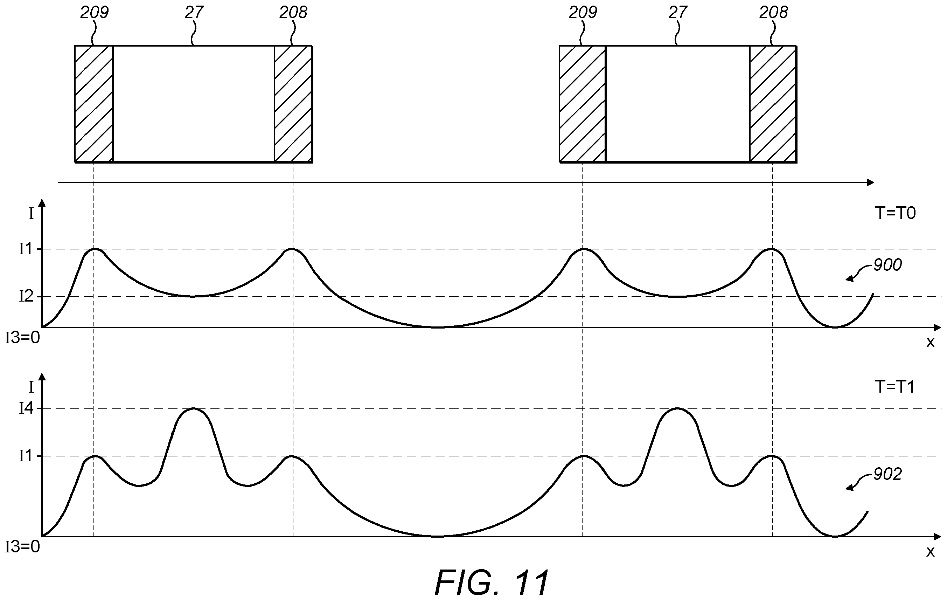

Comme montré sur la figure 11, le dispositif optique 30 permet d'éclairer différentes dernières cuvettes 24 localisées au sein de différentes barrettes d'analyse 20 placées sur différents rails 12. La figure 11 montre deux deuxièmes surfaces S2 relatives à deux dernières cuvettes 24. Compte tenu de la nature du matériau de la dernière cuvette 24, la deuxième surface S2 comprenant les bordures 208, 209 et la paroi 27 émet des signaux de fluorescence en réaction aux faisceaux d'excitation reçus sur la première surface S1. Dans la mesure où les bordures 208 et 209 ont une épaisseur supérieure à celle de la paroi 27, les signaux de fluorescence émis par les bordures 208 et 209 ont une intensité supérieure à l'intensité des signaux de fluorescence émis par la paroi 27.

Un dispositif de traitement du signal (non montré) localisé dans le dispositif d'analyse 1 et connecté au dispositif optique 30 permet de calculer différentes données relatives aux signaux de fluorescence émis par les bordures 208, 209 et la paroi 27, telles que l'unité de fluorescence relative (RFU).

Lors du déroulement du procédé d'analyse des échantillons à tester localisés dans les dernières cuvettes 24 correspondantes, différentes étapes se succèdent.

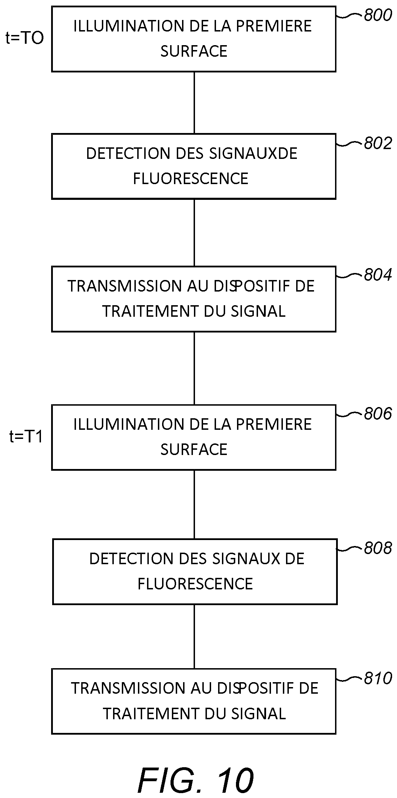

Ainsi, comme indiqué sur la figure 10, à un instant t=T0, le dispositif optique 30 procède à une étape 800 d'illumination sur la première surface S1 de la dernière cuvette 24, tandis que le dispositif optique 30 procède à une étape 802 de détection des signaux de fluorescence émis en provenance de la deuxième surface S2 de la dernière cuvette 24. Ces signaux de fluorescence ont une intensité relative à la valeur initiale de fluorescence de la dernière cuvette 24 illuminée et du milieu contenu dans la dernière cuvette 24 avant toute réaction avec l'échantillon à tester. Ainsi, à t=T0, le dispositif optique 30 permet de mesurer le blanc substrat, c'est-à-dire la mesure de la valeur initiale de fluorescence de la dernière cuvette 24 avant toute réaction associée à l'introduction du milieu de réaction.

Puis les signaux de fluorescence détectés sont transmis au dispositif de traitement du signal dans une étape 804. Le graphe 900 sur la figure 11 représente les variations d'intensité des signaux de fluorescence émis par la deuxième surface S2 comprenant les bordures 208, 209 et la paroi 27 en réponse à une illumination des deux dernières cuvettes 24, selon une émission des faisceaux lumineux comme montré sur la figure 9.

Sur le graphe 900, les signaux de fluorescence émis par les bordures 208 et 209 atteignent une intensité I1. La paroi 27 émet également un signal de fluorescence qui atteint une intensité I2 inférieure à l'intensité I1. Cette valeur de l'intensité I2 correspond à la valeur du signal de fluorescence émis au centre de la paroi 27.

On distingue généralement trois signaux de fluorescence en fonction de la localisation du faisceau d'excitation :
- le signal de fluorescence émis en réponse au faisceau d'excitation localisé à l'entrée de la paroi 26, c'est-à-dire à proximité d'une première bordure 207 ;
- le signal de fluorescence émis en réponse au faisceau d'excitation localisé au centre de la paroi 26 ;
- le signal de fluorescence émis en réponse au faisceau d'excitation localisé à la sortie de la paroi 26, c'est-à-dire à proximité d'une deuxième bordure 206.

Le dispositif de traitement du signal permet de déterminer la valeur de l'intensité du signal de fluorescence au centre de la paroi 27, c'est-à-dire au centre de la dernière cuvette 24.

Les bordures 208 et 209 ayant une épaisseur supérieure à l'épaisseur de la paroi 26, l'intensité I1 du signal de fluorescence émis par une bordure 208 ou 209 est supérieure à l'intensité I2 du signal de fluorescence émis par la paroi 27. Enfin, entre les deux dernières cuvettes 24, le signal de fluorescence atteint une intensité I3 inférieure à l'intensité I2. Ce signal de fluorescence correspond au bruit généré par les émissions parasites de particules localisées dans l'espace entre les deux dernières cuvettes 24. Ce signal de fluorescence d'intensité variable correspond au bruit de fond en l'absence de toute réaction immunologique. En l'absence de bruit de fond, la valeur de l'intensité I3 est égale à 0.

A un instant t=T1, le milieu de réaction obtenu après la séquence de réactions entre l'analyte potentiellement présent dans l'échantillon à tester et tous les réactifs et/ou solutions d'une ou plusieurs cuvettes 23 est disposé dans chacune des dernières cuvettes 24. Le milieu de réaction contient alors un produit de réaction produit par la combinaison des différentes réactions dues aux réactifs ou aux solutions en provenance des cuvettes 23. Deux de ces dernières cuvettes 24 sont représentées sur la figure 11. Comme indiqué sur la figure 11, à t=T1, le dispositif optique 30 procède alors à une étape d'illumination 806 et à une étape de détection 808 similaires aux étapes 800 et 802 comme indiqué lors de l'instant t=T0. Puis, les signaux de fluorescence émis sont transmis au dispositif de traitement du signal dans une étape 810, similaire à l'étape 804.

Le signal 902 sur la figure 11 représente les variations d'intensité des signaux de fluorescence émis en réponse à une illumination des deux dernières cuvettes 24, selon une émission des faisceaux d'excitation comme montré sur la figure 9.

Le signal 902 montre que les signaux de fluorescence émis par les bordures 208 et 209 atteignent une intensité I1 sensiblement égale à l'intensité I1 mesurée sur le signal 900. De manière similaire, le signal de fluorescence émis dans l'espace situé entre les deux dernières cuvettes 24 atteint une intensité I3 strictement égale à l'intensité I3 mesurée sur le signal. Cette intensité I3 correspond à une valeur nulle de fluorescence des deux dernières cuvettes. Le signal de fluorescence en provenance de la paroi 27 comprend le signal de fluorescence émis tant par la première surface S1 comprenant les bordures 206 et 207 et la paroi 26, que par la deuxième surface S2 comprenant les bordures 208 et 209 et la paroi 27, et le signal de fluorescence émis par le milieu de réaction en fonction de la quantité d'analyte présent dans l'échantillon à tester.

La somme de ces signaux de fluorescence combinés produit une intensité I4 supérieure à l'intensité I2 mesurée sur le signal 900 si l'analyte est présent dans l'échantillon à tester. On obtient ainsi une mesure de la quantité de fluorescence émise en fin de réaction pour une analyse spécifique définie par unité de fluorescence relative (RFU).

Le dispositif de traitement du signal procède à une soustraction du signal 900 et du signal 902. La différence obtenue entre le signal 900 à t=T0 et le signal 902 à t=T1 permet de déterminer la valeur de la fluorescence provenant de l'analyte présent dans l'échantillon à tester. Ainsi, le dispositif de traitement du signal permet de déterminer avec précision la valeur de l'intensité relative au seul échantillon et plus précisément au produit de réaction, en l'absence de tout signal parasite tel que les signaux de fluorescence en provenance des bordures 208, 209 et des parois 27. Le produit de réaction produit un signal de fluorescence proportionnel ou inversement proportionnel à la concentration de ce produit de réaction au sein de l'échantillon. On obtient alors une mesure de la quantité de fluorescence relative (RFV : Related Fluorescence Value) représentative de cet échantillon.

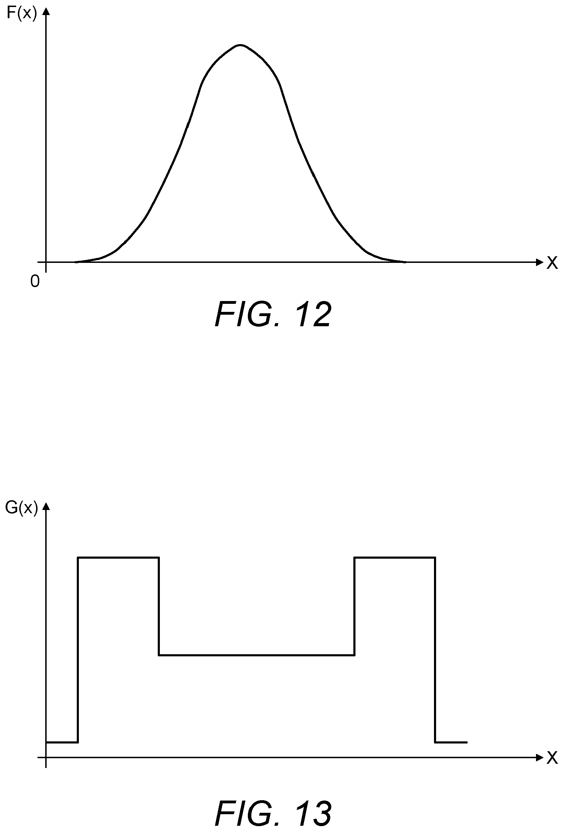

Alternativement, le dispositif de traitement du signal peut également réaliser une opération de déconvolution sur le signal 902. En effet, le profil d'éclairement F(x) de la source lumineuse 32 qui génère le faisceau d'excitation est tel que représenté sur la figure 12. De plus, le profil du signal de fluorescence G(x) généré par les bordures 208, 209, et la paroi 27 de la cuvette 24 dans la situation où elles sont éclairées par une source ponctuelle est tel que représenté sur la figure 13.

A t=T0, le dispositif optique 30 détecte donc un signal 900 qui représente le produit de convolution du signal généré par la source lumineuse 32 tel que sur la figure 12 et du profil d'émission de fluorescence de la cuvette tel que sur la figure 13. Ainsi lorsque le dispositif optique 30 à t=T1 détecte le signal tel que représenté par le graphe 902, le signal représente le produit de convolution du profil d'émission de fluorescence de la cuvette et du profil d'émission de fluorescence de l'analyte présent dans l'échantillon au sein du milieu de réaction.

Le dispositif de traitement du signal peut également appliquer une opération de déconvolution sur le signal 902 afin d'obtenir le profil d'émission de fluorescence généré par la paroi 27 de la cuvette et l'analyte présent dans l'échantillon à tester. En l'absence d'une opération de déconvolution, le signal de fluorescence utile exploitable est restreint à une localisation qui correspond au milieu de la paroi 27 de la dernière cuvette 24. En effet, le profil du signal de fluorescence utile exploitable est altéré par la largeur du faisceau d'excitation comme le montre le profil 900 de la figure 11. En revanche, la réalisation d'une opération de déconvolution du signal 902 par le profil d'éclairement F(x) de la source lumineuse permet d'extraire le signal de fluorescence produit uniquement par les bordures 208 et 209 de la dernière cuvette 24. Ainsi, la largeur du signal de fluorescence utile exploitable est augmentée. En utilisant les profils résultant de la même opération de déconvolution à t=T0 et à t=T1, il est possible de supprimer la contribution des bordures 208 et 209 du signal 902 mesuré.

Aussi, selon un autre objet de l'invention, le procédé de l'invention met en oeuvre une opération de déconvolution du profil d'émission de fluorescence, généré par une cuvette, par le profil d'éclairement d'une source lumineuse, c'est-à-dire par la distribution spatiale d'intensité lumineuse.

La présente invention permet de résoudre également les problèmes de réglage de position du dispositif optique 30 par rapport aux différentes barrettes d'analyse 20. En effet, l'utilisateur n'a plus besoin de positionner correctement le dispositif optique afin que la source lumineuse 32 illumine spécifiquement la première surface S1 en fonction de la largeur du faisceau d'excitation.

La présente invention permet aussi d'améliorer la sensibilité de lecture dans la mesure où les différents signaux produits permettent de déterminer la nature des signaux de fluorescence détectés. Ainsi, l'utilisateur peut aisément dissocier les signaux de fluorescence en provenance de la cuvette 24 des signaux de fluorescence en provenance du milieu de réaction comprenant le produit de réaction. Ainsi, il apparait qu'avec un faisceau d'excitation de largeur réduite tel que le faisceau d'une diode électroluminescente, la présente invention permet de reproduire les effets d'un faisceau d'excitation large d'une source lumineuse telle qu'une lampe xénon tout en évitant les inconvénients d'une telle source à faisceau d'excitation large.

En utilisant la déconvolution, la présente invention permet d'augmenter l'élargissement de la zone utile du signal de fluorescence lors de l'exploitation des signaux de fluorescence mesurés. Ainsi, des signaux de fluorescence générés par des éléments parasites tels que des bulles et/ou des artéfacts présents au sein de la cuvette considérée ne sont pas pris en compte pour l'analyse de l'échantillon.

## Revendications

1. Procédé d'analyse d'un échantillon à tester pour la détermination de la présence ou la quantification d'un analyte susceptible d'être présent dans ledit échantillon, mettant en oeuvre une réaction produisant un milieu de réaction issu dudit échantillon et possédant des propriétés de fluorescence, ledit milieu de réaction étant localisé au sein d'une cuvette (24), ledit milieu de réaction et ladite cuvette (24) formant un ensemble d'analyse possédant des propriétés fluorescentes en réponse à une illumination d'une source lumineuse (32) produisant un signal lumineux, ladite source lumineuse (32) étant mobile le long d'une surface S1 (26, 206, 207) comprenant une première bordure (206), une paroi (26) et une deuxième bordure (207) de la cuvette (24), ledit procédé comprenant les étapes suivantes :
- illumination de la surface S1 (26, 206, 207) de la cuvette (24) au moyen de la source lumineuse (32), avant introduction du milieu de réaction dans la cuvette (24) à partir de n positions différentes incluant les positions relatives à la première bordure (206), à la paroi (26) et à la deuxième bordure (207) pour la source lumineuse (32) ;
- détection d'un signal de fluorescence émis par la cuvette (24), pour chaque position n différente incluant les positions relatives à la première bordure (206), à la paroi (26) et à la deuxième bordure (207) pour la source lumineuse (32), en réponse à l'illumination et avant introduction du milieu de réaction dans la cuvette (32) pour produire un premier signal (900) ;
- illumination de l'ensemble d'analyse au moyen de la source lumineuse (32), après introduction du milieu de réaction dans la cuvette (24), à partir des n positions différentes incluant les positions relatives à la première bordure (206), à la paroi (26) et à la deuxième bordure (207) pour la source lumineuse (32) ;
- détection d'un signal de fluorescence émis par l'ensemble d'analyse, pour chaque position n différente incluant les positions relatives à la première bordure (206), à la paroi (26) et à la deuxième bordure (207) pour la source lumineuse (32), en réponse à l'illumination et après introduction du milieu de réaction dans la cuvette (24) pour produire un deuxième signal (902) ;
- réalisation d'une opération de calcul comprenant le premier signal (900) représentant les variations d'intensité des signaux de fluorescence détectés pour chaque position n différente incluant les positions relatives à la première bordure (206), à la paroi (26) et à la deuxième bordure (207) pour la source lumineuse (32), en réponse à l'illumination et avant introduction du milieu de réaction dans la cuvette (24) et le deuxième signal (902) pour chaque position n différente incluant les positions relatives à la première bordure (206), à la paroi (26) et à la deuxième bordure (207) pour la source lumineuse (32), en réponse à l'illumination et après introduction du milieu de réaction dans la cuvette (24) pour produire un signal résultant pour chaque position n différente incluant les positions relatives à la première bordure (206), à la paroi (26) et à la deuxième bordure (207) et correspondant à l'émission du signal de fluorescence produit uniquement par le milieu de réaction.

2. Procédé d'analyse selon la revendication 1, dans lequel l'illumination de la cuvette (24) comprend une illumination dans l'air, c'est-à-dire avant la première bordure (207) adjacente à la paroi (26) de la cuvette (24), sur la première bordure (207) adjacente à la paroi (26) de la cuvette (24), sur la paroi (26) de la cuvette (24), sur la seconde bordure (206) adjacente à la paroi (26) de la cuvette (24) et dans l'air, c'est-à-dire après la seconde bordure (206) adjacente à la paroi (26) de la cuvette (24).

3. Procédé d'analyse selon la revendication 1 ou 2, dans lequel la détection d'un signal de fluorescence comprend la détection d'un signal de fluorescence en provenance d'une deuxième surface S2 (27, 208, 209) de la cuvette (24), opposée à la surface S1 (26, 206, 207) comprenant une première bordure (206), une paroi (26) et une deuxième bordure (207).

4. Procédé d'analyse selon l'une des revendications précédentes, dans lequel le signal résultant correspond à un signal de fluorescence émis par l'analyte.

5. Procédé d'analyse selon l'une des revendications précédentes, dans lequel l'intensité du signal résultant est proportionnelle ou inversement proportionnelle à la concentration de l'analyte dans l'échantillon testé.

6. Procédé d'analyse selon l'une des revendications précédentes, dans lequel la source lumineuse (32) comprend une diode électroluminescente (LED).

7. Procédé d'analyse selon l'une des revendications précédentes dans lequel la réaction est une réaction antigène-anticorps, en particulier par dosage immuno-enzymatique.

8. Procédé d'analyse selon l'une des revendications précédentes, dans lequel l'étape de réalisation d'une opération de calcul comprend la réalisation d'une opération de déconvolution du signal résultant par le profil d'éclairement de la source lumineuse (32) pour produire un signal résultant correspondant à l'émission du signal de fluorescence produit uniquement par le milieu de réaction.

9. Système d'analyse d'un échantillon à tester pour la détermination de la présence ou la quantification d'un analyte susceptible d'être présent dans ledit échantillon, mettant en oeuvre une réaction produisant un milieu de réaction issu dudit échantillon et possédant des propriétés de fluorescence, ledit milieu de réaction étant localisé au sein d'une cuvette (24), ledit milieu de réaction et ladite cuvette (24) formant un ensemble d'analyse possédant des propriétés de fluorescence, ledit système comprenant :
- un dispositif optique (30) comprenant une source lumineuse (32) mobile le long d'une surface S1 (26, 206, 207) comprenant une première bordure (206), une paroi (26) et une deuxième bordure (207) de la cuvette (24) pour illuminer l'ensemble d'analyse au moyen de faisceaux d'excitation pour chaque position n différente incluant les positions relatives à une première bordure (206), à une paroi (26) et à une deuxième bordure (207) de la cuvette (24), et un dispositif de détection (40) pour détecter les émissions de fluorescence émises par l'ensemble d'analyse pour chaque position n différente incluant les positions relatives à une première bordure (206), à une paroi (26) et à une deuxième bordure (207) de la cuvette (24),
- un dispositif de traitement du signal pour réaliser une opération de calcul sur un premier signal (900) représentant les variations d'intensité des signaux de fluorescence détectés pour chaque position n différente incluant les positions relatives à la première bordure (206), à la paroi (26) et à la deuxième bordure (207) pour la source lumineuse (32), en réponse à l'illumination et avant introduction du milieu de réaction dans la cuvette (24) et un deuxième signal (902) pour chaque position n différente incluant les positions relatives à la première bordure (206), à la paroi (26) et à la deuxième bordure (207) pour la source lumineuse (32), en réponse à l'illumination et après introduction du milieu de réaction dans la cuvette (24) pour produire un signal résultant pour chaque position n différente incluant les positions relatives à la première bordure (206), à la paroi (26) et à la deuxième bordure (207) et correspondant à l'émission du signal de fluorescence produit uniquement par le milieu de réaction.

10. Système d'analyse selon la revendication 9, dans lequel le dispositif optique (30) est adapté pour illuminer la surface S1 (26, 206, 207) comprenant une première bordure (206), une paroi (26) et une deuxième bordure (207) de la cuvette (24).

11. Système d'analyse selon la revendication 9 ou 10, dans lequel le dispositif optique (30) détecte les émissions de fluorescence en provenance d'une deuxième surface S2 (27, 208, 209) de la cuvette (24), opposée à la surface S1 (26, 206, 207) comprenant une première bordure (206), une paroi (26) et une deuxième bordure (207).

## Patentansprüche

1. Ein Analyseverfahren einer zu testenden Probe zur Bestimmung des Vorliegens oder zur Quantifizierung eines Analyten, der in der Probe vorliegen kann, wobei eine Reaktion durchgeführt wird, die ein Reaktionsmedium aus der Probe erzeugt und Fluoreszenzeigenschaften aufweist, wobei sich das Reaktionsmedium in einer Küvette (24) befindet, wobei das Reaktionsmedium und die Küvette (24) eine Analyseanordnung bilden, die als Reaktion auf eine Beleuchtung durch eine Lichtquelle (32), die ein Lichtsignal erzeugt, Fluoreszenzeigenschaften aufweist, wobei die Lichtquelle (32) entlang einer Oberfläche S1 (26, 206, 207), die einen ersten Rand (206), eine Wand (26) und einen zweiten Rand (207) der Küvette (24) beinhaltet, beweglich ist, wobei das Verfahren die folgenden Schritte beinhaltet:
- Beleuchten der Oberfläche S1 (26, 206, 207) der Küvette (24) mittels der Lichtquelle (32) vor dem Einführen des Reaktionsmediums in die Küvette (24), ausgehend von unterschiedlichen Positionen n einschließlich der Positionen relativ zu dem ersten Rand (206), der Wand (26) und dem zweiten Rand (207) für die Lichtquelle (32);
- Nachweisen eines Fluoreszenzsignals für jede unterschiedliche Position n, einschließlich der Positionen relativ zu dem ersten Rand (206), der Wand (26) und dem zweiten Rand (207) für die Lichtquelle (32), das von der Küvette (24) als Reaktion auf die Beleuchtung und vor dem Einführen des Reaktionsmediums in die Küvette (32) emittiert wird, um ein erstes Signal (900) zu erzeugen;
- Beleuchten der Analyseanordnung mittels der Lichtquelle (32) nach dem Einführen des Reaktionsmediums in die Küvette (24), ausgehend von den unterschiedlichen Positionen n, einschließlich der Positionen relativ zu dem ersten Rand (206), der Wand (26) und dem zweiten Rand (207) für die Lichtquelle (32);
- Nachweisen eines Fluoreszenzsignals, das von der Analyseanordnung, für jede unterschiedliche Position n, einschließlich der Positionen relativ zu dem ersten Rand (206), der Wand (26) und dem zweiten Rand (207) für die Lichtquelle (32) als Reaktion auf die Beleuchtung und nach dem Einführen des Reaktionsmediums in die Küvette (24) emittiert wird, um ein zweites Signal (902) zu erzeugen;
- Ausführen einer Rechenoperation, beinhaltend das erste Signal (900), das die Intensitätsschwankungen der nachgewiesenen Fluoreszenzsignale für jede unterschiedliche Position n, einschließlich der Positionen relativ zu dem ersten Rand (206), der Wand (26) und dem zweiten Rand (207), für die Lichtquelle (32), als Reaktion auf die Beleuchtung und vor dem Einführen des Reaktionsmediums in die Küvette (24) darstellt, und das zweite Signal (902) für jede unterschiedliche Position n, einschließlich der Positionen relativ zu dem ersten Rand (206), der Wand (26) und dem zweiten Rand (207) für die Lichtquelle (32), als Reaktion auf die Beleuchtung und nach dem Einführen des Reaktionsmediums in die Küvette (24), um ein Signal zu erzeugen, das für jede unterschiedliche Position n resultiert, einschließlich der Positionen relativ zu dem ersten Rand (206), der Wand (26) und dem zweiten Rand (207) und der Emission des Fluoreszenzsignals entspricht, das ausschließlich von dem Reaktionsmedium erzeugt wird.

2. Analyseverfahren gemäß Anspruch 1, wobei die Beleuchtung der Küvette (24) eine Beleuchtung in der Luft beinhaltet, das heißt vor dem an die Wand (26) der Küvette (24) angrenzenden ersten Rand (207), an dem an die Wand (26) der Küvette (24) angrenzenden ersten Rand (207), auf der Wand (26) der Küvette (24), an dem an die Wand (26) der Küvette (24) angrenzenden zweiten Rand (206) und in der Luft, das heißt nach dem an die Wand (26) der Küvette (24) angrenzenden zweiten Rand (206).

3. Analyseverfahren gemäß Anspruch 1 oder 2, wobei das Nachweisen eines Fluoreszenzsignals das Nachweisen eines Fluoreszenzsignals von einer zweiten Oberfläche S2 (27, 208, 209) der Küvette (24) beinhaltet, die der Oberfläche S1 (26, 206, 207), die einen ersten Rand (206), eine Wand (26) und einen zweiten Rand (207) beinhaltet, gegenüberliegt.

4. Analyseverfahren gemäß einem der vorhergehenden Ansprüche, wobei das resultierende Signal einem Fluoreszenzsignal entspricht, das von dem Analyten emittiert wird.

5. Analyseverfahren gemäß einem der vorhergehenden Ansprüche, wobei die Intensität des resultierenden Signals zu der Konzentration des Analyten in der getesteten Probe proportional oder umgekehrt proportional ist.

6. Analyseverfahren gemäß einem der vorhergehenden Ansprüche, wobei die Lichtquelle (32) eine Leuchtdiode (LED) beinhaltet.

7. Analyseverfahren gemäß einem der vorhergehenden Ansprüche, wobei die Reaktion eine Antigen-Antikörper-Reaktion, insbesondere durch Enzymimmunoassay, ist.

8. Analyseverfahren gemäß einem der vorhergehenden Ansprüche, wobei der Schritt des Ausführens einer Rechenoperation das Ausführen einer Dekonvolutionsoperation des Signals beinhaltet, das durch das Bestrahlungsprofil der Lichtquelle (32) resultiert, um ein resultierendes Signal zu erzeugen, das der Emission des Fluoreszenzsignals entspricht, das ausschließlich durch das Reaktionsmedium erzeugt wird.

9. Ein Analysesystem einer zu testenden Probe zur Bestimmung des Vorliegens oder zur Quantifizierung eines Analyten, der in der Probe vorliegen kann, wobei eine Reaktion durchgeführt wird, die aus der Probe ein Reaktionsmedium erzeugt, und Fluoreszenzeigenschaften aufweist, wobei das Reaktionsmedium sich in einer Küvette (24) befindet, wobei das Reaktionsmedium und die Küvette (24) eine Analyseanordnung bilden, die Fluoreszenzeigenschaften aufweist, wobei das System Folgendes beinhaltet:
- eine optische Vorrichtung (30), beinhaltend eine Lichtquelle (32), die entlang einer Oberfläche S1 (26, 206, 207), beinhaltend einen ersten Rand (206), eine Wand (26) und einen zweiten Rand (207) der Küvette (24) zum Beleuchten der Analyseanordnung mittels Anregungsstrahlen für jede unterschiedliche Position n, einschließlich der Positionen relativ zu einem ersten Rand (206), einer Wand (26) und einem zweiten Rand (207) der Küvette (24), beweglich ist, und eine Nachweisvorrichtung (40) zum Nachweisen der Fluoreszenzemissionen, die von der Analyseanordnung für jede unterschiedliche Position n, einschließlich der Positionen relativ zu einem ersten Rand (206), einer Wand und einem zweiten Rand (207) der Küvette (24), emittiert wird,
- eine Signalverarbeitungsvorrichtung zum Ausführen einer Rechenoperation an einem ersten Signal (900), das die Intensitätsschwankungen der nachgewiesenen Fluoreszenzsignale für jede unterschiedliche Position n, einschließlich der Positionen relativ zu dem ersten Rand (206), der Wand (26) und dem zweiten Rand (207) für die Lichtquelle (32), als Reaktion auf die Beleuchtung und vordem Einführen des Reaktionsmediums in die Küvette (24) darstellt, und einem zweiten Signal (902) für jede unterschiedliche Position n, einschließlich der Positionen relativ zu dem ersten Rand (206), der Wand (26) und dem zweiten Rand (207) für die Lichtquelle (32), als Reaktion auf die Beleuchtung und nach dem Einführen des Reaktionsmediums in die Küvette (24), um ein Signal zu erzeugen, das für jede unterschiedliche Position n resultiert, einschließlich der Positionen relativ zu dem ersten Rand (206), der Wand (26) und dem zweiten Rand (207) und der Emission des Fluoreszenzsignals entspricht, das ausschließlich von dem Reaktionsmedium erzeugt wird.

10. Analysesystem gemäß Anspruch 9, wobei die optische Vorrichtung (30) angepasst ist, um die Oberfläche S1 (26, 206, 207), beinhaltend einen ersten Rand (206), eine Wand (26) und einen zweiten Rand (207) der Küvette (24), zu beleuchten.

11. Analysesystem gemäß Anspruch 9 oder 10, wobei die optische Vorrichtung (30) Fluoreszenzemissionen von einer zweiten Oberfläche S2 (27, 208, 209) der Küvette (24), der Oberfläche S1 (26, 206, 207), die einen ersten Rand (206), eine Wand (26) und einen zweiten Rand (207) beinhaltet gegenüberliegend, nachweist.

## Claims

1. A method of analysing a sample to be tested to determine the presence of, or to quantify, an analyte capable of being present in said sample, employing a reaction which produces a reaction medium derived from said sample and which possesses fluorescence properties, said reaction medium being located within a well (24), said reaction medium and said well (24) forming an analysis assembly which possesses fluorescent properties in response to an illumination from a light source (32) which produces a light signal, said light source (32) being moveable along a surface S1 (26, 206, 207) comprising a first edge (206), a wall (26) and a second edge (207) of the well (24), said method comprising the following steps:
- illumination of the surface S1 (26, 206, 207) of the well (24) by means of the light source (32), before introduction of the reaction medium into the well (24) from n different positions including the positions relative to the first edge (206), to the wall (26) and to the second edge (207) for the light source (32);
- detection of a fluorescence signal emitted by the well (24), for each different position n including the positions relative to the first edge (206), to the wall (26) and to the second edge (207) for the light source (32), in response to the illumination and before introduction of the reaction medium into the well (32) to produce a first signal (900);
- illumination of the analysis assembly by means of the light source (32), after introduction of the reaction medium into the well (24), from n different positions including the positions relative to the first edge (206), to the wall (26) and to the second edge (207) for the light source (32);
- detection of a fluorescence signal emitted by the analysis assembly, for each different position n including the positions relative to the first edge (206), to the wall (26) and to the second edge (207) for the light source (32), in response to the illumination and after introduction of the reaction medium into the well (24) to produce a second signal (902);
- performing a calculation operation comprising the first signal (900) representing the variations in intensity of the fluorescence signals detected for each different position n including the positions relative to the first edge (206), to the wall (26) and to the second edge (207) for the light source (32), in response to the illumination and before introduction of the reaction medium into the well (24) and the second signal (902) for each different position n including the positions relative to the first edge (206), to the wall (26) and to the second edge (207) for the light source (32), in response to the illumination and after introduction of the reaction medium into the well (24) to produce a resulting signal for each different position n including the positions relative to the first edge (206), to the wall (26) and to the second edge (207) and corresponding to the emission of the fluorescence signal produced solely by the reaction medium.

2. The analysis method according to Claim 1, wherein the illumination of the well (24) comprises an illumination in the air, i.e. before the first edge (207) adjacent to the wall (26) of the well (24), on the first edge (207) adjacent to the wall (26) of the well (24), on the wall (26) of the well (24), on the second edge (206) adjacent to the wall (26) of the well (24) and in the air, i.e. after the second edge (206) adjacent to the wall (26) of the well (24).

3. The analysis method according to Claim 1 or 2, wherein the detection of a fluorescence signal comprises the detection of a fluorescence signal coming from a second surface S2 (27, 208, 209) of the well (24), opposite the surface S1 (26, 206, 207) comprising a first edge (206), a wall (26) and a second edge (207).

4. The analysis method according to any one of the preceding claims, wherein the resulting signal corresponds to a fluorescence signal emitted by the analyte.

5. The analysis method according to any one of the preceding claims, wherein the intensity of the resulting signal is proportional or inversely proportional to the concentration of the analyte in the sample tested.

6. The analysis method according to any one of the preceding claims, wherein the light source (32) comprises a light-emitting diode (LED).

7. The analysis method according to any one of the preceding claims, wherein the reaction is an antigen-antibody reaction, in particular by immunoenzyme assay.

8. The analysis method according to any one of the preceding claims, wherein the step of performing a calculation operation comprises performing an operation of deconvoluting the resulting signal by the lighting profile of the light source (32) to produce a resulting signal corresponding to the emission of the fluorescence signal produced solely by the reaction medium.

9. A system of analysing a sample to be tested to determine the presence of, or to quantify, an analyte capable of being present in said sample, employing a reaction which produces a reaction medium derived from said sample and which possesses fluorescence properties, said reaction medium being located within a well (24), said reaction medium and said well (24) forming an analysis assembly which possesses fluorescence properties, said system comprising:
- an optical device (30) comprising a light source (32) moveable along a surface S1 (26, 206, 207) comprising a first edge (206), a wall (26) and a second edge (207) of the well (24) to illuminate the analysis assembly by means of excitation beams for each different position n including the positions relative to a first edge (206), to a wall (26) and to a second edge (207) of the well (24), and a detection device (40) to detect the fluorescence emissions emitted by the analysis assembly for each different position n including the positions relative to a first edge (206), to a wall (26) and to a second edge (207) of the well (24),
- a device for processing the signal to perform a calculation operation on a first signal (900) representing the variations in intensity of the fluorescence signals detected for each different position n including the positions relative to the first edge (206), to the wall (26) and to the second edge (207) for the light source (32), in response to the illumination and before introduction of the reaction medium into the well (24) and a second signal (902) for each different position n including the positions relative to the first edge (206), to the wall (26) and to the second edge (207) for the light source (32), in response to the illumination and after introduction of the reaction medium into the well (24) to produce a resulting signal for each different position n including the positions relative to the first edge (206), to the wall (26) and to the second edge (207) and corresponding to the emission of the fluorescence signal produced solely by the reaction medium.

10. The analysis system according to Claim 9, wherein the optical device (30) is suitable for illuminating the surface S1 (26, 206, 207) comprising a first edge (206), a wall (26) and a second edge (207) of the well (24).

11. The analysis system according to Claim 9 or 10, wherein the optical device (30) detects the fluorescence emissions coming from a second surface S2 (27, 208, 209) of the well (24), opposite the surface S1 (26, 206, 207) comprising a first edge (206), a wall (26) and a second edge (207).
